(19) Europäisches Patentamt
European Patent Office
Office européen des brevets

(11) EP 0 991 709 B1

# (12) EUROPÄISCHE PATENTSCHRIFT

(45) Veröffentlichungstag und Bekanntmachung des Hinweises auf die Patenterteilung:
**09.11.2005 Patentblatt 2005/45**

(21) Anmeldenummer: **98930746.7**

(22) Anmeldetag: **28.05.1998**

(51) Int Cl.[7]: **C08K 5/3475**, C08K 5/54, C08J 7/04, C08L 69/00

(86) Internationale Anmeldenummer:
**PCT/EP1998/003156**

(87) Internationale Veröffentlichungsnummer:
**WO 1998/056852 (17.12.1998 Gazette 1998/50)**

## (54) UV-STABILISATOREN FÜR SILOXAN-SYSTEME

UV-STABILIZERS FOR SILOXANE SYSTEMS

AGENTS ANTI U.V. POUR SYSTEMES A BASE DE SILOXANE

(84) Benannte Vertragsstaaten:
**AT BE CH DE DK ES FR GB IT LI NL**

(30) Priorität: **10.06.1997 DE 19724396**

(43) Veröffentlichungstag der Anmeldung:
**12.04.2000 Patentblatt 2000/15**

(73) Patentinhaber: **Bayer MaterialScience AG**
**51368 Leverkusen (DE)**

(72) Erfinder: **BIER, Peter**
**D-47800 Krefeld (DE)**

(56) Entgegenhaltungen:
**EP-A- 0 732 355**
**DE-A- 2 810 072**
**US-A- 5 556 936**
**WO-A-93/15063**
**US-A- 4 051 161**

Bemerkungen:
Die Akte enthält technische Angaben, die nach dem Eingang der Anmeldung eingereicht wurden und die nicht in dieser Patentschrift enthalten sind.

EP 0 991 709 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft nicht-flüchtige, UV-stabilisierende Mischungen für Siloxan-Lacksysteme, deren UV-stabilisierende Wirkstruktur bestimmte Hydroxybenztriazole sind und die daher besonders für die UV-Stabilisierung von Thermoplasten, insbesondere von aromatischen Polycarbonaten geeignet sind.

Um Materialien vor schädlichen Umwelteinflüssen zu schützen, werden diese häufig mit einer schützenden Oberfläche versehen. Als besonders geeignet haben sich dabei Lacke auf Siloxanbasis erwiesen, die den Materialien u.a. eine kratzfeste Oberfläche verleihen.

Diese Lacke können sogenannte UV-stabilisierende Substanzen enthalten, um den Lack selbst und das darunterliegende Material, das sogenannte Substrat, gegen schädliche UV-Strahlen zu schützen. Neben einem über eine lange Zeit wirkenden UV-Schutz wird von diesen Substanzen unter anderem gefordert, daß sie nicht flüchtig sind, so daß sie in der Lackschicht homogen verteilt bleiben und weder beim Aushärten noch beim späteren Einsatz des Lackes aus der Lackschicht austreten. Ferner dürfen sich die UV-stabilisierenden Substanzen nicht schnell zersetzen, müssen sich mit den Lacken dauerhaft homogen mischen und der die UV-stabilisierende Substanzen enthaltende Lack sollte transparent sein.

EP-A 732 355 offenbart Formkörper, die eine UV-Schutzschicht aus einem Polymeren, welches Hydroyxbenzotriazole enthält, aufweisen.

US 5,556,936 offenbart Polycarbonate, welche chemisch gebundene Hydroyxbenzotriazole als UV-Schutz enthalten.

DE-A 28 10 072 offenbart ein Beschichtungssystem auf Silanbasis, dem unter anderem auch UV-Absorber zugesetzt werden können.

Die US-A 4 278 804 und die US-A 4 051 161 beziehen sich auf UV-stabilisierende Wirksubstanzen und Lacke, die diese enthalten. Die dort offenbarten Substanzen haben jedoch den Nachteil, daß ihr UV-Schutz unzureichend ist, daß sie sich zu schnell zersetzen und/oder daß das die Stabilisatoren enthaltende Siloxan-System gelbstichig ist.

Aus der US-A 5 438 142 ist femer die UV-stabilisierende Wirksubstanz, 1-(3'-(Benzotriazol-2''-yl)-4'-hydroxyphenyl)-1,1-bis(4-hydroxyphenyl)ethan bekannt. Diese Wirksubstanz hat jedoch den Nachteil, daß sie sich mit Lacken auf Siloxanbasis nicht dauerhaft mischen läßt.

Es stellt sich deshalb die Aufgabe, ein UV-Stabilisatorsystem zur Verfügung zu stellen, das die oben geschilderten Nachteile nicht aufweist.

Die Aufgabe wird erfindungsgemäß durch die Bereitstellung von UV-stabilisierenden Mischungen gelöst, enthaltend Hydroxybenztriazol der nachstehenden allgemeinen Formel (1) und Epoxidgruppen aufweisende, hydrolysierbare Silane.

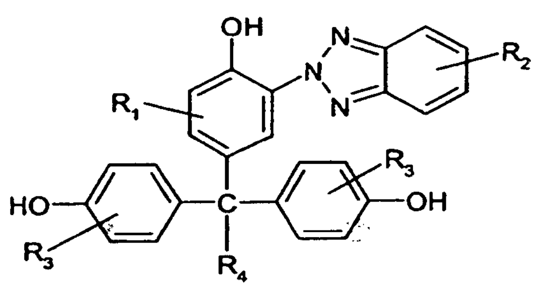

(1)

$R_1$: H, $C_1$-$C_{18}$-Alkyl, $C_5$-$C_6$-Cycloalkyl oder $C_6$-$C_{12}$-Aryl,

$R_2$: H, Halogen, vorzugsweise Cl oder $C_1$-$C_{12}$-Alkyl,

$R_3$: H, $C_1$-$C_{12}$-Alkyl, Halogen, vorzugsweise Cl oder Br oder $C_6$-$C_{12}$-Aryl,

$R_4$: H, $C_1$-$C_{18}$-Alkyl oder $C_6$-$C_{18}$-Aryl.

Gegenstand der vorliegenden Erfindung sind ferner UV-stabilisierende Mischungen mit einem molaren Verhältnis von Epoxidgruppen des Silanes zum Hydroxybenztriazol der allgemeinen Formel (1), das größer als 2,5, vorzugsweise größer als 4, besonders bevorzugt größer als 16 ist. Das molare Verhältnis von Epoxid-Einheit des Silanes zum Hydroxybenztriazol der allgemeinen Formel (1) sollte jedoch 200:1 nicht überschreiten.

Die erfindungsgemäßen Mischungen sind zur UV-Stabilisierung von Siloxan-Systemen, insbesondere von

kratz- und abriebfesten Siloxanbeschichtungsmaterialien geeignet. Solche UV-stabilisierten Beschichtungsmaterialien, vorzugsweise Lacke, können zur Beschichtung von Materialien aller Art wie z.B. Holz, Textilien, Papier, Steinwaren, vorzugsweise jedoch zur Beschichtung von Kunststoffen, Metallen, Glas und Keramik, besonders bevorzugt zur Beschichtung von Thermoplasten und ganz besonders bevorzugt zur Beschichtung von Polycarbonaten verwendet werden

Die für die erfindungsgemäßen nicht flüchtigen UV-stabilisierenden Mischungen verwendeten Hydroxybenztriazole sind Verbindungen der allgemeinen Formel (1).

Bevorzugt wird jedoch 1-(3'-(Benzotriazol-2"-yl)-4'-Hydroxyphenyl)-1,1-bis(4-hydroxyphenyl)ethan - im folgenden mit THPE-BZT abgekürzt - eingesetzt. Die Herstellung von THPE-BZT ist detailliert in der US-A 5 438 142 beschrieben, wobei der allgemeine Syntheseweg im Schema 1 dieser Patentschrift offenbart wird. Das Produkt ist kommerziell von der Firma Hoechst/Celanese zu beziehen.

Allgemein werden unter Epoxidgruppen enthaltenden Silanen Verbindungen verstanden, die zum einen mindestens einen Epoxidring besitzen und gleichzeitig Gruppen aufweisen, die unter Hydrolysebedingungen Silanol-Strukturen bilden.

Epoxisilane, wie sie erfindungsgemäß bevorzugt verwendet werden, sind z.B. in US-A 2 946 701 beschrieben. Sie sind Verbindungen der allgemeinen Formeln (2) oder (3):

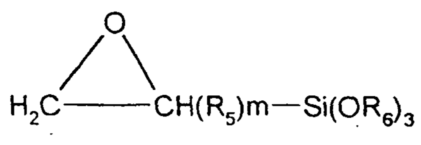

(2)

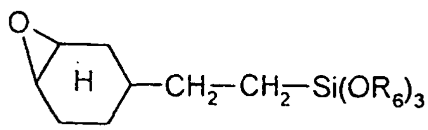

(3)

$R_5$ ist ein zweiwertiger Kohlenwasserstoffrest mit höchstens 9 Kohlen- stoffatomen oder ein zweiwertiger Rest mit höchstens 9 Kohlenstoffatomen, bestehend aus C, H und O-Atomen, wobei das O-Atom als Etherbindungs- rest vorliegt. Vorzugsweise ist
$R_5$ = - $CH_2OCH_2CH_2CH_2$ -.

$R_6$ ist ein aliphatischer Kohlenwasserstoffrest mit höchstens 4 Kohlenstoffatomen, ein Acylrest mit höchstens 4 Kohlenstoffatomen oder ein Rest der Formel $(CH_2CH_2O)_nZ$, bei dem n mindestens 1 ist und Z ein aliphatischer Kohlenwas- serstoffrest mit höchstens 4 Kohlenwasserstoffatomen bedeutet;

m ist 0 oder 1.

Die Herstellung dieser Epoxisilane ist ebenfalls in der US-A 2 946 701 beschrieben. Dieses Patent wird deshalb als Referenz eingeführt. Besonders bevorzugte Epoxisilane sind solche Verbindungen, in denen $R_6$ = Methyl oder Ethyl ist. Sie sind kommerziell erhältlich, u. a. von den Firmen Union-Carbide und Hüls AG als:

| | |
|---|---|
| A-187 bzw. Dynasilan Glymo | 3-Glycidyloxypropyltrimethoxysilan |
| A-186 | 2-(3,4-Epoxycyclohexyl)ethyltrimethoxysilan |

<u>Herstellung der UV-stabilisierenden Mischungen</u>

Die Herstellung der UV-stabilisierenden Mischungen erfolgt durch ein homogenes Vermischen von Hydroxybenztriazol der allgemeinen Formel 1 mit den Epoxidgruppen enthaltenden, hydrolysierbaren Silanen und Erhitzen dieser Mischung. Das Erhitzen sollte mindestens 30 min bei mindestens 90°C erfolgen. Vorzugsweise sollte dabei die Temperatur oberhalb 120°C liegen.

Als besonders günstig hat sich ein Mischungsverhältnis herausgestellt, bei dem stöchiometrisch mehr Epoxidgruppen vorhanden sind als von den beiden freien für die UV-Stabilisierung nicht erforderlichen phenolischen OH-Gruppen der Hydroxybenztriazole der allgemeinen Formel (1). Das molare Verhältnis von Epoxid-Einheit des Silans zu Hydroxybenztriazol der allgemeinen Formel 1 sollte daher größer als 2,5, vorzugsweise größer als 4, besonders bevorzugt größer als 16 sein.

Die UV-stabilisierenden Komponenten müssen nicht unbedingt separat hergestellt werden, um dann dem zu stabilisierenden Siloxansystem hinzugesetzt zu werden, sondern können auch während der Synthese der Siloxan-Systeme/Siloxanbeschichtungsmaterialien als Teilschritt in situ synthetisiert werden.

Siloxan-Systeme / Siloxanbeschichtungsmaterialien

Die Siloxansysteme sind im wesentlichen thermisch aushärtende Systeme, die vorzugsweise durch Kondensationsreaktion zu -Si-O-Si-Verknüpfungen vernetzen. Parallel dazu können auch andere Vernetzungsmechanismen ablaufen. Derartige Systeme sind z. B. in US-A 3 790 527, 3 865 755, 3 887 514, 4 243 720, 4 278 804, 4 680 232, 4 006 271, 4 476 281, in DE-A 4 011 045, 4 122 743, 4 020 316, 3 917 535, 3 706 714, 3 407 087, 3 836 815, 2 914 427, 3 135 241, 3 134 777, 3 100 532, 3 151 350, in DE-A 3 005 541, 3 014 411, 2 834 606, 2 947 879, 3 016 021, 2 914 427 und 4 338 361 beschrieben und sind als Gegenstand der vorliegenden Offenbarung zu betrachten.

Ein weiterer Gegenstand der Erfindung sind erfindungsgemäß UV-stabilisierte Siloxansysteme.

Bevorzugt werden Siloxansysteme eingesetzt, die teilchenförmiges Material enthalten, das aus Oxiden, Oxidhydraten, Nitriden und Carbiden von Si, Al, Sb und B sowie von Übergansmetallen, vorzugsweise Ti, Ce, Fe und Zr, ausgewählt ist und eine Teilchengröße im Bereich von 1 bis 100 nm, vorzugsweise 2 bis 50 nm aufweist.

Dem Siloxansystem soll soviel der erfindungsgemäßen UV-stabilisierenden Mischung bezogen auf den Feststoff des Siloxansystems zugegeben werden, daß der Anteil an Hydroxybenztriazol bezogen auf den Feststoff des Siloxansystems 0,3 bis 20, vorzugsweise 3 bis 15, besonders bevorzugt 5 bis 10 Gew.-% beträgt.

Für die Herstellung von kratzfesten Beschichtungssystemen und deren Komponenten auf Siloxanbasis wird durch Referenz auf die DE-A 2 914 427 und DE-A 4 338 361 verwiesen, die damit Gegenstand der vorliegenden Beschreibung sind.

Substrate, Materialien

Die mit den erfindungsgemäßen UV-stabilisierenden Mischung versehenen Siloxansysteme können als Bulkmaterialien sowie als Beschichtungsmaterialien verwendet werden. Die Auswahl der Substratmaterialien zur Beschichtung ist uneingeschränkt. Vorzugsweise eignen sich diese UV-stabilisierten Beschichtungsmaterialien zur Beschichtung von Holz, Textilien, Papier, Steinwaren, Metallen, Glas, Keramik und Kunststoffen und dabei besonders zur Beschichtung von Thermoplasten, wie sie in Becker/Braun, Kunststoffhandbuch, Carl Hanser Verlag, München, Wien, 1992 beschrieben sind Ganz besonders geeignet sind sie für die Beschichtung von transparenten Thermoplasten und dabei vorzugsweise von Polycarbonaten.

Für Beschichtungszwecke werden die üblichen Beschichtungsverfahren angewandt, z.B. Tauchen, Fluten, Gießen, Schleudern, Spritzen oder Aufstreichen.

Die Beschichtung wird in Schichtdicken von z.B. 2 bis 200 µm, vorzugsweise 2 bis 30 µm und besonders bevorzugt 5 bis 15 µm aufgetragen. Gegebenenfalls kann das Substrat vor der Aufbringung der Beschichtung mit einer Haftvermittler- oder Primerschicht grundiert werden.

Die Aushärtung der Lacke erfolgt vorzugsweise bei Temperaturen >90°C.

Thermoplastische, aromatische Polycarbonate im Sinne der vorliegenden Erfindung sind sowohl Homopolycarbonate als auch Copolycarbonate, die Polycarbonate können in bekannter Weise linear oder verzweigt sein.

Ein Teil, bis zu 80 Mol-%, vorzugsweise von 20 Mol-%, bis zu 50 Mol-% der Carbonat-Gruppen in den geeigneten Polycarbonaten können durch aromatische Dicarbonsäureester-Gruppen ersetzt sein. Derartige Polycarbonate, die sowohl Säurereste der Kohlensäure als auch Säurereste von aromatischen Dicarbonsäuren in die Molekülkette eingebaut enthalten, sind, genau bezeichnet, aromatische Polyestercarbonate. Sie sollen unter dem Oberbegriff der thermoplastischen, aromatischen Polycarbonate subsummiert werden.

Einzelheiten der Herstellung von Polycarbonaten sind in Hunderten von Patentschriften seit etwa 40 Jahren niedergelegt. Beispielhaft sei hier nur auf "Schnell, Chemistry and Physics of Polycarbonates", Polymer Reviews, Volume 9, Interscience Publishers, New York, London, Sydney 1964, auf D.C. PREVORSEK, B.T. DEBONA and Y. KESTEN, Corporate Research Center, Allied Chemical Corporation, Morristown, New Jersey 07960, "Synthesis of Poly(ester Carbonate) Copolymers" in Journal of Polymer Science, Polymer Chemistry Edition, Vol. 19,75-90 (1980), auf D. Freitag, U. Grigo, P. R. Müller, N. Nouvertne', BAYER AG, "Polycarbonates" in Encyclopedia of Polymer Science and Engineering, Volume 11, Second Edition, 1988, Seiten 648-718 und schließlich auf Dres. U. Grigo, K. Kircher und P. R. Müller "Polycarbonate" in Becker /Braun, Kunststoff-Handbuch, Band 3/1, Polycarbonate, Polyacetale, Polyester, Celluloseester, Carl Hanser Verlag München, Wien, 1992, Seiten 117-299 verwiesen.

Die thermoplastischen Polycarbonate haben mittlere Molekulargewichte $\overline{M}_w$ (ermittelt durch Messung der relativen Viskosität bei 25°C in $CH_2Cl_2$ und einer Konzentration von 0,5 g pro 100 ml $CH_2Cl_2$) von 12 000 bis 400 000, vorzugsweise von 18 000 bis 80 000 und insbesondere von 22 000 bis 60 000.

Gegenstand der vorliegenden Erfindung sind demnach auch beschichtete Materialien, vorzugsweise Polycarbonat und besonders bevorzugt kratzfest beschichtetes Polycarbonat.

**Beispiele**

**Beispiel 1:**

a) UV-stabiliserende Mischung aus THPE-BZT und 3-Glycidyloxy-propyltrimethoxysilan (Glymo)

50 g THPE-BZT und 450 g 3-Glycidyloxypropyltrimethoxysilan werden vorgelegt und unter Stickstoff-Beschleierung und Rühren auf 140 bis 150°C aufgeheizt und bei dieser Temperatur eine Stunde gehalten.

Folgende Variationen wurden von dieser Mischung (Mischung 1) durchgeführt:

Mischung:

| | | |
|---|---|---|
| 2 | 100 g THPE-BZT | 400 g Glymo |
| 3 | 150 g " | 350 g " |
| 4 | 200 g " | 300 g " |
| 5 | 100 g THPE-BZT | 400 g α-(3,4-Epoxycyclohexyl)ethyl trimethoxysilan |

b) Herstellung des Siloxanbeschichtungsmaterials nach DE-OS 2 914 427 (Beschichtungssol I)

α) Zu 300 g kolloidaler Kieselsäure mit 30 Gew.-% $SiO_2$-Gehalt werden 19,8 g Eisessig, 210 g destilliertes Wasser und 227 g Isopropanol gegeben. Nach gründlicher Durchmischung werden 900 g Methyltriethoxysilan zugesetzt und die Mischung unter Rühren auf 60°C erwärmt. Man beläßt die Mischung 4 Stunden lang bei dieser Temperatur und fügt anschließend weitere 1200 g Isopropanol zur Mischung hinzu. Nach Abkühlen des Produkts auf Raumtemperatur wird die schwach opake Lösung filtriert.

β) In einem mit Rührer und Rückflußkühler versehenen Gefäß werden 340 g Isopropanol, 190 g Tetraethoxysilan und 360 g Methyltriethoxysilan vorgelegt. Dieses Gemisch wird mit 180 g 0,05 n Salzsäure versetzt und zur Durchführung der Cohydrolyse fünf Stunden unter Rückfluß erwärmt. Nach der Umsetzung wird das Gemisch auf Raumtemperatur abgekühlt. Es resultiert eine Lösung, die ein Teilhydrolysat von Tetraethoxysilan (5,1 %, berechnet als $SiO_2$) und Teilhydrolysat von Methyltriethoxysilan (12,6 %, berechnet als $CH_3SiO_{1,5}$) enthält.

Vor Verwendung als Beschichtungsmaterial werden die beiden Komponenten α) und β) im Verhältnis 1:1 miteinander vermischt und in einer Mischung aus 60 Gewichtsteilen n-Butanol, 40 Gew.-Teilen Essigsäure und 20 Gew.-Teilen Toluol gelöst.

c) Herstellung eines Siloxanbeschichtungsmaterials nach DE-OS 4 338 361 (Beschichtungssol II)

Zur Herstellung eines Böhmitsols wurden 12,82 g Essigsäure-stabilisiertes (6,4 Gew.-% Essigsäure) Böhmitpulver mit 104,62 g 0,1 n HCl versetzt. Durch anschließende Ultraschall-Behandlung (20 Minuten) entsteht eine transparente, farblose Lösung, von der 24,3 g mit einer Mischung aus 118,17 g GPTS (3 Glycidyloxypropyl-trimethoxy-silan) und 62,50 g TEOS (Tetraethyl Orthosilicat) versetzt wurde. Die Reaktionsmischung wurde 2 Stunden lang bei Raumtemperatur gerührt und anschließend unter Eiskühlung mit 18,93 g Aluminiumtributoxyethanolat versetzt. Das resultierende klare Sol wurde 2 Stunden bei Raumtemperatur gerührt und dann unter Eiskühlung mit 93,14 g des obigen Böhmitsols und 79,30 g Butoxyethanol versetzt.

d) UV-stabilisierte Beschichtungssole I und II

Es wurden zu jeweils 1 000 g des Beschichtungssoles I und II jeweils 75 g der erfindungsgemäßen UV-stabilisierenden Mischung 2 hinzugegeben. Mit diesen Zusammensetzungen wurden Quarzgläser beschichtet und die UV-Lichttransmission mit einem Beckmann DU 70 Fotometer im Wellenlängenbereich von 250 bis 600 nm gemessen. Die Schichtdicke betrug 5 μm und absorbierte >98% der für Polycarbonat kritischen Strahlung mit einer Wellenlänge <350 nm.

e) Beschichten von Substraten und Prüfung der Eigenschaften der Beschichtungen

Platten aus Polycarbonat auf Basis Bisphenol A (Tg = 147°C, $\overline{M}_w$ 27 500) mit den Maßen 105 x 150 x 4 mm wurden mit Isopropanol gereinigt und durch Tauchen in eine Mischung aus 3 Gew.-% Aminopropyltrimethoxysilan und 97 Gew.-% Butylglykol mit anschließender 0,5-stündiger Temperaturbehandlung bei 130°C geprimert. Anschließend wurden die Platten jeweils in Beschichtungssole I oder II bei einer Tauchgeschwindigkeit V = 100 cm/min mit einer Lackschicht von 20 μm beschichtet. Nach 10 min Ablüften bei Raumtemperatur wurden die beschichteten Platten 1 h bei 130°C getrocknet. Die Schichtdicke der Kratzfestlacke betrug nach Trocknung ca. 5 μm. Die beschichteten Platten wurden nach erfolgter Aushärtung 2 Tage bei Raumtemperatur gelagert und dann einer definierten UV-Belichtung unterzogen.

UV-Belichtunesprüfung

Die Beanspruchung der lackierten Polycarbonatplatten erfolgte durch gefilterte Xenonbogenstrahlung mit einem Beregnungszyklus gemäß DIN 53387-1-A-X unter folgenden Prüfbedingungen:

| Bewitterungsgerät | Xenon - WOM |
|---|---|
| Bestrahlungsstärke bei 340 nm | 0,35 W / $m^2$ (vorzugsweise) |
| Filterkombination | Innen: Pyrex Außen: Pyrex |
| Schwarztafeltemperatur | 60°C ± 5°C |
| Schwarzstandardtemperatur | 65°C ± 3°C |
| Betriebsweise | Gleichlauf |
| Beregnungszyklus | 102 : 18 |
| Relative Luftfeuchte | 60 - 80 % |

Als Kriterium für die Witterungsstabilität der lackierten Platten wurde die Vergilbung in Abhängigkeit der Belichtungszeit herangezogen. Der entsprechende Gelbwert der Platten wurde nach ASTM D 1925 - 70 als Yellowness Index : Y.I. bestimmt.

Y.I.-Werte nach Xenon-WOM 102:18 Bewitterung

| Proben | 0 h | 1 000 h | 2 000 h | 3 000 h | 5 000 h |
|---|---|---|---|---|---|
| Polycarbonat mit UV-stabil. Beschichtungssol I gemäß Mischung 2 | 2,1 | 2,3 | 2,7 | 2,9 | 4,3 |
| Polycarbonat mit UV-stabil. Beschichtungssol II gemäß Mischung 2 | 2,4 | 2,6 | 3,1 | 3,2 | 4,9 |
| **Vergleich** | | | | | |
| Polycarbonat gemäß Beispiel le mit Beschichtungssol II ohne UV-Stabilisierung | 1,9 | 2,6 | 6,3 a) | 7,9 a) | - b) |

a) Risse, Delaminierung der Lackschicht.

b) Keine Lackschicht mehr vorhanden.

**Patentansprüche**

1. UV-Stabilisator-Mischung, enthaltend

A) Hydroxybenztriazol gemäß der allgemeinen Formel (1):

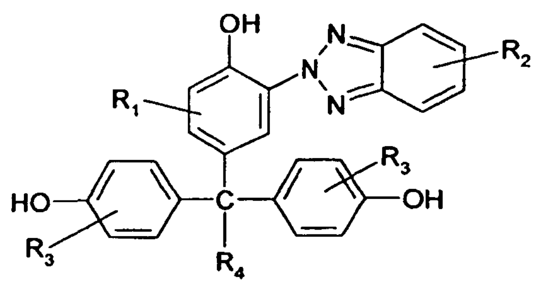

(1)

mit

$R_1$: H, $C_1$-$C_{18}$-Alkyl, $C_5$-$C_6$-Cycloalkyl oder $C_6$-$C_{12}$-Aryl,

$R_2$: H, Halogen oder $C_1$-$C_{12}$-Alkyl,

$R_3$: H, $C_1$-$C_{12}$-Alkyl, Halogen, vorzugsweise Cl oder Br oder $C_6$-$C_{12}$-Aryl,

$R_4$: H, $C_1$-$C_{18}$-Alkyl oder $C_6$-$C_{18}$-Aryl

und

B) und Epoxidgruppen enthaltende, hydrolysierbare Silane,

wobei das molare Verhältnis von Epoxidgruppen des Silans zum Hydroxybenztriazol der allgemeinen Formel (1) größer als 2,5 ist.

2. UV-Stabilisator-Mischung gemäß Anspruch 1, **dadurch gekennzeichnet, daß** das Hydroxybenztriazol der allgemeinen Formel (1) ein 1-(3'-(benzotriazol-2''-yl)-4'-hydroxyphenyl)-1,1-bis(4-hydroxyphenyl)ethan ist.

3. Verfahren zur Herstellung der UV-Stabilisator-Mischung gemäß einem der Ansprüche 1 oder 2, **dadurch gekennzeichnet, daß** man Hydroxybenztriazole der Formel (1) und die Epoxidgruppen enthaltenden, hydrolysierbaren Silane vermischt und mindestens 30 min auf mindestens 90°C erhitzt.

4. Siloxan-System enthaltend eine UV-Stabilisator-Mischung erhalten gemäß Anspruch 3, **dadurch gekennzeichnet, daß** der Anteil an Hydroxybenztriazol bezogen auf den Feststoff des Siloxansystems 0,3 bis 20 beträgt.

5. Siloxanbeschichtungsmaterial, enthaltend ein Siloxansystem gemäß Anspruch 4 und gegebenenfalls teilchenförmiges Material, bestehend aus einer Verbindung ausgewählt aus der Gruppe gebildet aus Oxiden, Oxidhydraten, Nitriden und Carbiden von Si, Al, Sb und B sowie von Übergangsmetallen, vorzugsweise Ti, Ce, Fe und Zr, wobei das Material eine Teilchengröße im Bereich von 1 bis 100 nm aufweist.

6. Verwendung des Siloxanbeschichtungsmaterials gemäß Anspruch 5 zur Beschichtung von Substratmaterialien jeglicher Art, vorzugsweise von Thermoplasten, besonders bevorzugt von Polycarbonaten.

7. Beschichtetes Material, vorzugsweise Polycarbonat, **dadurch gekennzeichnet, daß** zumindestens die der Strahlung ausgesetzten Oberflächen des Materials mit einem Siloxanbeschichtungsmaterial gemäß Anspruch 5 beschichtet ist.

**Claims**

1. UV-stabiliser mixture containing

A) hydroxybenzotriazole according to the general formula (1) :

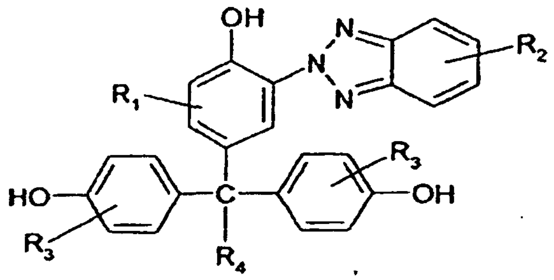

(1)

where:

$R_1$ : H, $C_1$-$C_{18}$ alkyl, $C_5$-$C_6$ cycloalkyl or $C_6$-$C_{12}$ aryl,

$R_2$: H, halogen or $C_1$-$C_{12}$ alkyl ,

$R_3$: H, $C_1$-$C_{12}$ alkyl, halogen, preferably Cl or Br, or $C_6$-$C_{12}$ aryl,

$R_4$: H, $C_1$-$C_{18}$ alkyl or $C_6$-$C_{18}$ aryl

and

B) hydrolysable silanes containing epoxy groups,

wherein the molar ratio of epoxy groups of the silane to the hydroxybenzotriazole of the general formula (1) is greater than 2.5.

2. UV-stabiliser mixture according to claim 1, **characterised in that** the hydroxybenzotriazole of the general formula (1) is a 1-(3'-(benzotriazol-2''-yl)-4'-hydroxyphenyl)-1,1-bis(4-hydroxyphenyl)ethane.

3. Process for the production of the UV-stabiliser mixture according to one of claims 1 or 2, **characterised in that** hydroxybenzotriazoles of the formula (1) and the hydrolysable silanes containing epoxy groups are mixed and heated to at least 90°C for at least 30 minutes.

4. Siloxane system containing a UV-stabiliser mixture obtained according to claim 3, **characterised in that** the proportion of hydroxybenzotriazole relative to the solids content of the siloxane system is 0.3 to 20.

5. Siloxane coating material containing a siloxane system according to claim 4 and optionally particulate material consisting of a compound selected from the group comprising oxides; oxide hydrates, nitrides and carbides of Si, Al, Sb and B and of transition metals, preferably Ti, Ce, Fe and Zr, wherein the material has a particle size in the range from 1 to 100 nm.

6. Use of the siloxane coating material according to claim 5 for coating substrate materials of any kind, preferably thermoplastics, particularly preferably polycarbonates.

7. Coated material, preferably polycarbonate, **characterised in that** at least the surfaces of the material exposed to radiation are coated with a siloxane coating material according to claim 5.

## Revendications

1. Mélange stabilisant contre les UV, contenant

A) un hydroxybenzotriazole de formule générale (1)

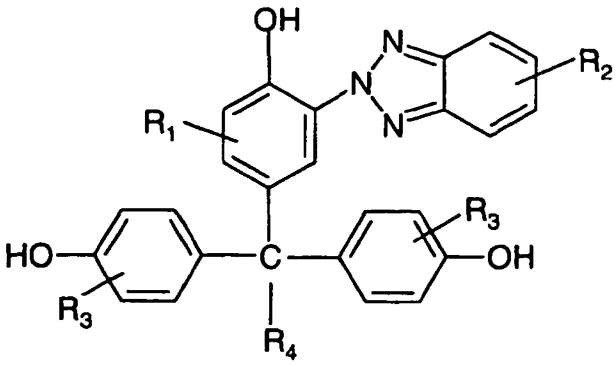

(1)

dans laquelle

$R_1$ = H, alkyle en $C_1$-$C_{18}$, cycloalkyle en $C_5$-$C_6$ ou aryle en $C_6$-$C_{12}$,
$R_2$ = H, halogène ou alkyle en $C_1$-$C_{12}$,
$R_3$ = H, alkyle en $C_1$-$C_{12}$, halogène, de préférence Cl ou Br, ou aryle en $C_6$-$C_{12}$,
$R_4$ = H, alkyle en $C_1$-$C_{18}$ ou aryle en $C_6$-$C_{18}$,

et
B) des silanes hydrolysables contenant des groupes époxyde,

avec un rapport molaire des groupes époxyde du silane à l'hydroxybenzotriazole de formule générale (1) qui est supérieur à 2,5.

2. Mélange stabilisant contre les UV selon la revendication 1, **caractérisé en ce que** l'hydroxybenzotriazole de formule générale (1) est le 1-[3'-(benzotriazol-2"-yl)-4'-hydroxyphényl]-1,1-bis(4-hydroxyphényl)-éthane.

3. Procédé pour la préparation du mélange stabilisant contre les UV selon une des revendications 1 ou 2, **caractérisé en ce que** l'on mélange les hydroxybenzotriazoles de formule (1) et les silanes hydrolysables à groupes époxyde et on chauffe pendant au moins 30 min à au moins 90°C.

4. Composition de siloxanes contenant un mélange stabilisant contre les UV obtenu selon la revendication 3, **caractérisée en ce que** la proportion de l'hydroxybenzotriazole, par rapport aux matières solides de la composition de siloxanes est de 0,3 à 20.

5. Produit de revêtement à base de siloxanes contenant une composition de siloxanes selon la revendication 4 et le cas échéant une matière à l'état de particules consistant en un composé choisi parmi les oxydes, oxydes hydratés, nitrures et carbures de Si, Al, Sb et B, et les métaux de transition, de préférence Ti, Ce, Fe et Zr, ladite matière ayant une dimension de particule dans l'intervalle de 1 à 100 nm.

6. Utilisation du produit de revêtement à base de siloxanes selon la revendication 5 pour le revêtement de substrats de tous types, de préférence de résines thermoplastiques et plus spécialement de polycarbonates.

7. Matériau revêtu, de préférence polycarbonate, **caractérisé en ce que**, au moins sur la surface du matériau exposée au rayonnement, il porte un revêtement d'un produit de revêtement à base de siloxanes selon la revendication 5.